# EUROPEAN PATENT APPLICATION

(11) **EP 4 272 766 A1**
(43) Date of publication of application: **08.11.2023**
(21) Application number: 22171291.2
(22) Date of filing: 03.05.2022
(51) Int. Cl.: A61K 49/00

(54) **DIAGNOSTIC CHEWING GUM FOR CHRONIC DISEASES**

(71) Applicant: Dianox ApS, 2100 København Ø (DK)
(72) Inventor: CODE, Christian Sean, 5000 Odense C (DK); KONJEN, Huram, 2620 Albertslund (DK)
(74) Representative: Zacco Denmark A/S

(57) **Abstract**

A diagnostic chewing gum for detecting biomarkers of chronic diseases via oral fluids (viz. volatile or small organic metabolites). The gum stimulates the release of biomarkers in sufficient quantities for detection, and comprises color changing liposomes specific for the biomarker of interest. A polymer within the liposomes exhibits a color change that is visible with the naked eye.

## Description

### Technical field of the invention

The present invention relates to the field of medical diagnosis, in particular to a diagnostic chewing gum and its use for the diagnosis of a chronic disease.

### Background of the invention

In the medical field, biofluids are typically subjected to a range of chemical and/or biochemical analyses to provide data in the diagnosis of a disease or ailment. Many of these analyses often require samples obtained from a venous blood drawn at a clinic.

US8257940B2 describes a gum for screening diabetes.

EP2823050B1 describes a gum for screening pathogens.

US6866863B2 describes ingestibles possessing intrinsic color change.

US9523683B2 describes functionalized polydiacetylene sensors.

US7776371B2 describes methods and compositions for preparing consumables with optical shifting properties.

There is a medical need for a point-of-need sensor for rapid testing of biofluids from the oral cavity to determinate the presence of specific biomarkers relating to chronic diseases.

It is an object of the present invention to provide a diagnostic product for enabling rapid testing of biofluids from the oral cavity to determinate the presence of specific biomarkers relating to chronic diseases. It is also an object of the present invention to provide such a diagnostic product for enabling a rapid, non-invasive, and inexpensive screening method, which enables fast adoption for the broad screening of populations.

### Summary of the invention

In a first aspect the present invention provides a diagnostic chewing gum having a reporter element for identifying or quantifying a volatile or small organic metabolite in a biofluid, comprising:
I. a gum base material; and
II. at least one sweetener or flavor such as a sugar, dextran, xylitol, aspartame, a flavor from peppermint, spearmint, wintergreen or cinnamon; and
III. a reporter material comprising a polymer, such as polydiacetylene, with or without a lipid that upon contact with a specific volatile or small organic metabolite from said biofluid generates a color change that is visible with the naked eye.

In a second aspect, the present invention provides the use of a diagnostic chewing gum with at least one flavor according to the second aspect for stimulating the oral cavity, namely the salivary glands, and for providing fresh breath and reducing dental caries.

In a third aspect, the present invention provides the use of a diagnostic chewing gum according to the first aspect as a sink for concentrating the volatile or small organic metabolites.

In a fourth aspect, the present invention provides the use of a diagnostic chewing gum according to the first aspect for detecting volatile or small organic metabolites for screening of chronic diseases from a group containing: ammonia, acetone, pentane, or uric acid.

In a fifth aspect, the present invention provides the use of a diagnostic chewing gum according to the third aspect for rapid reporting of a color change in the presence of a chronic disease.

### Brief description of the drawings

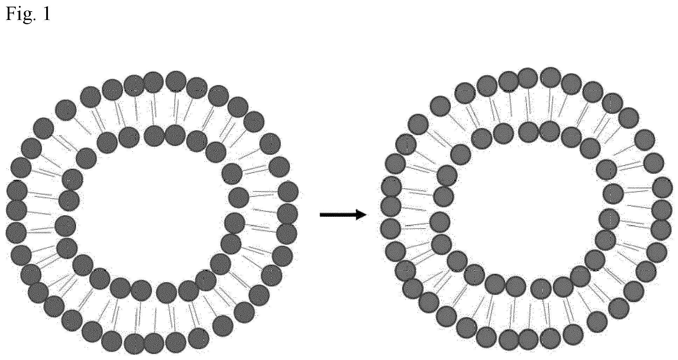

Fig. 1 Shows a polydiacetylene reporter embedded in a lipid nanoparticle (1) upon addition of a metabolite (2) the lipid nanoparticle changes color as a result of the polydiacetylene reporter embedded in the lipid nanoparticle (3). Lipid nanoparticles containing different reporter molecules and/or lipid elements can change the sensitivity of the reporter.

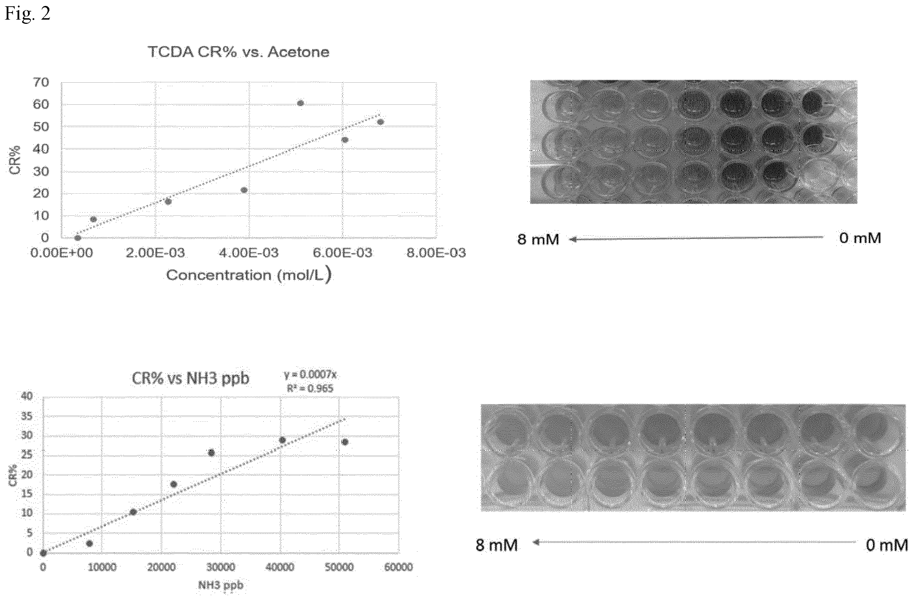

Fig. 2 Shows a graph of the %CR (Percent Color Responsiveness) of a polydiacetylene reporter dependent on the concentration of different volatile or small organic molecules.

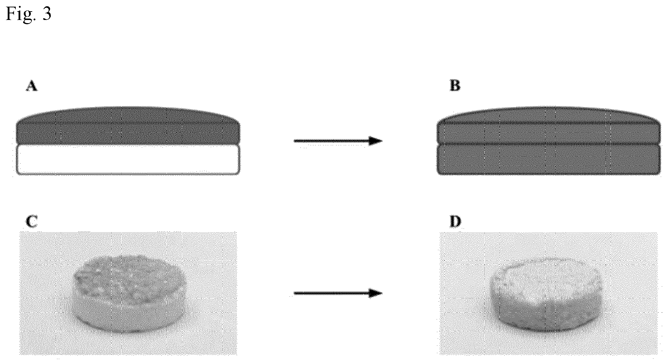

Fig. 3 Shows an illustration of the gum with the polydiacetylene liposomes present on the upper layer of the gum base (A). After the gum comes into contact with a volatile or small organic metabolite from the oral cavity, the color is dispersed with the whole gum appearing red (B). A photo of the gum before the addition of a volatile or small organic metabolite (C). A photo of the gum after the addition of a volatile or small organic metabolite (D).

### Description

The present invention provides a diagnostic chewing gum having a reporter element for identifying or quantifying a volatile or small organic metabolite in a biofluid, comprising:
I. a gum base material; and
II. at least one sweetener or flavorant such as a sugar, dextran, xylitol, aspartame, a flavor from peppermint, spearmint, wintergreen or cinnamon; and
III. a reporter material comprising a polymer, such as polydiacetylene, with or without a lipid that upon contact with a specific volatile or small organic metabolite from said biofluid generates a color change that is visible with the naked eye.

A volatile or small organic metabolite is a substance, e.g. (ammonia, acetone, pentane, or uric acid) that is traceable in specific biofluids, e.g. blood, saliva, urine, breath, and other biological samples. Non-limiting examples of volatile or small organic metabolites are ammonia, acetone, pentane and uric acid.

Analysis of volatile or small organic metabolites may require specific devices like gas chromatography or liquid chromatography coupled with mass spectrometry to determine the concentration. In the present invention is provided a diagnostic chewing gum comprising a chewing gum base that can specifically absorb and concentrate a metabolite of interest before it evaporates in air or degrades.

The gum base component of the diagnostic chewing gum is an elastomer. Gum bases are typically made from three main components: Resin, wax, and elastomer. Resin such as terpene is the main chewable portion. Wax primarily serves to soften the gum.

A sweetener or a flavorant in the chewing gum can stimulate the oral cavity to produce more saliva, thus allowing more of the volatile or small organic metabolite to be concentrated over time.

In an embodiment the diagnostic chewing gum comprises a sweetener.

In a further embodiment the sweetener is a sugar, a sugar alcohol, aspartame, acesulfame K., sucralose, natural stevia or a mixture thereof.

In an embodiment the diagnostic chewing gum comprises a flavorant.

In a further embodiment the flavorant is selected from spearmint, peppermint, wintergreen and cinnamon.

The present invention provides a novel approach for detecting volatile or small organic metabolites relating to specific chronic diseases.

In an embodiment the chewing gum incorporates liposomes containing a reporter that changes its structure upon binding to a volatile or small organic metabolite, which exhibits a color change. This change is effected when a minimum metabolite concentration is reached. The minimum concentration of a volatile or small organic metabolite in biofluids generates a change in the lipid nanoparticles that is visible with the naked eye.

In a further embodiment the liposomes are lipid nanoparticles. Such lipid nanoparticles are typically embedded within the gum base and they can have a size in the range of 50 to about 3000 nm in which the particle contains a chromophoric polymer.

The chromophoric polymer changes color upon binding to a volatile or small organic metabolite. One example of a chromophoric polymer is polydiacetylene. Polydiacetylenes are a class of linear conjugated polymers derived from topochemical polymerization of diacetylene monomers.

**Table 1. Volatile or small organic metabolite concentrations present in the oral cavity**

| **Volatile or Small Organic Met abolite** | **Chronic Dise ase** | **Concentration Found in Saliva** | **Reference** |
|---|---|---|---|
| Ammonia | Chronic Kidney, Disease, Diabetic Nephropathy | 97-170 mg/l | Chen, Wen, et al. "Biochemical pathways of breath ammonia (NH3) generation in patients with end-stage renal disease undergoing hemodialysis." Journal of breath research 10.3 (2016): 036011. |
| Urea | Ulcer (H. pylori) | 10 ng/ml | Yu, Min, Xue-Yan Zhang, and Qing Yu. "Detection of oral Helicobacter Pylori infection using saliva test cassette." Pakistan Journal of Medical Sciences 31.5 (2015): 1192. |
| Acetone, Beta-hydroxybutarate | Ketoacidosis: Type 1 Diabetes, Type 2 Diabetes | 11.9-154.4 mg/l | Fujii, Shinya, et al. "Determination of acetone in saliva by reversed-phase liquid chromatography with fluorescence detection and the monitoring of diabetes mellitus patients with ketoacidosis." Clinica Chimica Acta 430 (2014): 140-144. |
| Isoprene | Lung Cancer | 2.882×10⁻⁸ | Chen, Xing, et al. "A study of the volatile organic compounds exhaled by lung cancer cells in vitro for breath diagnosis." Cancer: Interdisciplinary International Journal of the American Cancer Society 110.4 (2007): 835-844. |
| Uric Acid | Preeclampsia/Pregnan cy Induced Hypertension | 16.8-50.4 mg/l | Püschl, Ida Catharina, et al. "Salivary uric acid as a predictive test of preeclampsia, pregnancy-induced hypertension and preterm delivery: A pilot study." Acta Obstetricia et Gynecologica Scandinavica 99.10 (2020): 1339-1345. |
| Pentane; Carbon Disulfide | Schizophrenia | 25 pmol/l; 10 pmol/1 | Phillips, Michael, Michael Sabas, and Joel Greenberg. "Increased pentane and carbon disulfide in the breath of patients with schizophrenia." Journal of clinical pathology 46.9 (1993): 861-864. |

## Claims

1. A diagnostic chewing gum having a reporter element for identifying or quantifying a volatile or small organic metabolite in a biofluid, comprising:
IV. a gum base material; and
V. at least one sweetener or flavorant such as a sugar, dextran, xylitol, aspartame, a flavor from peppermint, spearmint, wintergreen or cinnamon; and
VI. a reporter material comprising a polymer, such as polydiacetylene, with or without a lipid that upon contact with a specific volatile or small organic metabolite from said biofluid generates a color change that is visible with the naked eye.

2. The diagnostic chewing gum according to claim 1, in which the gum base material allows the concentration of volatile or small organic metabolites in saliva to interact within its polymer matrix.

3. The diagnostic chewing gum according to claim 1, in which the flavor molecule increases the salival flow.

4. The diagnostic chewing gum according to claim 1, in which the flavor molecule provides fresh breath.

5. The diagnostic chewing gum according to claim 1, in which the reporter molecule is embedded in a liposome of size 50-3000 nm, and the liposomal particles can be crystallized in a carbohydrate.

6. The diagnostic chewing gum according to claim 1 and claim 2, in which the carbohydrate can crystallize and dry the liposomes.

7. The diagnostic chewing gum according to any of the preceding claims, wherein the chewing gum matrix can trap said volatile or small organic metabolites have a molecular weight between 10 g/mol to 300 g/mol.

8. The diagnostic chewing gum according to any of the preceding claims, wherein said volatile or small organic metabolites are biomarkers relating to a chronic disease.

9. The diagnostic chewing gum according to any of the preceding claims, wherein said volatile or small organic metabolites are for detecting or quantifying at least one of the following metabolites: Ammonia, Urea, Acetone, Beta-hydroxybutarate, Isoprene, Uric Acid, Pentane and Carbon Disulfide.

10. The diagnostic chewing gum according to any of the preceding claims, wherein said reporter material is a liposomal sensor comprising a polymerized polydiacetylene molecule, wherein the liposome exhibits a color change from blue to red (absorbance in wavelengths 450-740 nm) in the presence of a volatile or small organic metabolite.

11. The diagnostic chewing gum according to any of the preceding claims, wherein said biofluid is saliva.

12. The diagnostic chewing gum according to any of the preceding claims, wherein said shape, size, and tensile strength of the gum is designed to maximize salival flow.

13. The diagnostic chewing gum according to any of the preceding claims, wherein the material of the gum can help reduce dental caries in persons afflicted with chronic diseases.

14. Use of the diagnostic chewing gum as defined in any of claims 1-13 for providing bioanalytical data for testing an individual for having a chronic disease or for potentially having a chronic disease.

15. Use of the diagnostic chewing gum as defined in any of claims 1-13 for detecting or quantifying at least one of the following metabolites in the saliva of an individual: Ammonia, Urea, Acetone, Beta-hydroxybutarate, Isoprene, Uric Acid, Pentane and Carbon Disulfide.
